# EUROPEAN PATENT SPECIFICATION

(11) **EP 2 269 517 B1**
(45) Date of publication and mention of the grant of the patent: **18.09.2013**
(21) Application number: 09251702.8
(22) Date of filing: 01.07.2009
(51) Int. Cl.: A61B 17/00, A61M 5/28

(54) **Liquid applicator**
Flüssigkeitsapplikator
Applicateur de liquide

(43) Date of publication of application: 05.01.2011
(73) Proprietor: Advanced Medical Solutions (Plymouth) Limited, Plymouth Devon PL7 5BG (GB)
(72) Inventor: Stenton, Richard J., Horrabridge Devon PL20 7QZ (GB)
(74) Representative: Atkinson, Peter Birch

(56) References cited:
- WO-A-2008/001067
- US-A- 4 478 358
- US-A- 4 960 340
- US-A1- 2006 049 203
- US-B1- 6 409 406

## Description

The present invention relates to applicators for liquids, e.g. adhesives, sealants and coatings. The invention relates particularly (but not necessarily exclusively) to liquid applicators for use in applying medical adhesives, sealants and coatings to skin wounds. Applicators of the invention are intended particularly (but again not necessarily exclusively) to be of the single-use, disposable type.

The term liquid as used herein is intended to encompass gels and pastes.

Applicators for small to medium quantities of liquid medical adhesives, sealants and coatings (particularly those that polymerise on application, e.g. cyanoacrylates) tend to be based on mechanical rupture of a thin-walled glass or plastic ampoule contained within a flexible walled plastics housing. The squeezing of the applicator housing causes rupture of the ampoule thus allowing the liquid to be discharged through an outlet of the applicator.

Typical of the state of the art is the Duraprep^{™} applicator made by 3M whereby a lever is depressed causing deformation and breakage of an internal ampoule thereby releasing the enclosed fluid. A further applicator that incorporates levers for fracturing an internal ampoule is disclosed in WO 2008/001067 A. Similarly the DERMABOND^{™} applicator made by James Alexander consists of a flexible tube which is compressed between thumb and forefinger to crush a thin-walled glass ampoule and release the fluid for dispensing. The applicators incorporate a porous component which is intended to allow discharge of the liquid but retain pieces of fractured glass within the applicator. However these applicators nevertheless can expose the user or patient to the risk of contact with broken glass.

US 4 478 358 A discloses an applicator comprising a resiliently squeezable body providing a reservoir for holding a liquid to be dispensed by the applicator and a cap through which liquid is dispensed *via* a mouth of the body. A diaphragm is provided across the mount of the body and an elongate spindle extends from the cap, through an aperture in the diaphragm into the reservoir. The applicator is provided with an applicator arrangement such that squeezing the walls of the body portion causes the spindle to enlarge in the aperture in the diaphragm and allow liquid to be discharged from the reservoir to the cap assembly.

It is therefore an object of the present invention to obviate or mitigate the above mentioned disadvantages.

According to the present invention there is provided a liquid applicator comprising:
(a) a hollow body portion incorporating (i) a reservoir portion for containing a liquid to be applied by the applicator, and (ii) an outlet portion having first and second ends, the first end being connected to an outlet mouth of the reservoir portion,
(b) an applicator tip assembly provided on the second end of the outlet portion for discharge of liquid from the reservoir portion *via* said outlet portion,
(c) a plug located in the mouth of the reservoir portion, and
(d) actuator means for squeezing the walls of the reservoir portion to effect discharge of liquid from the reservoir portion *via* the outlet portion and the applicator tip assembly, wherein
said plug and mouth arc configured so that the plug is moveable towards the applicator tip assembly from a first position at which it seals the reservoir portion to a second position at which fluid may follow out of the reservoir portion, characterised in that the actuator means are adapted to apply an actuating force to the mouth wall to effect movement of the plug from its first position to the second position.

In the applicator of the invention, therefore, the liquid to be dispensed is retained in a reservoir that is scaled by a plug until usage. The first actuator formations on the applicator arc capable of dislodging the plug so as to create a path to allow liquid to flow out of the reservoir portion. Subsequently the second actuator formations are employed to squeeze the walls of the reservoir portion and effect discharge of the liquid. Thus the applicator of the invention avoids the use of a glass ampoule and the attendant possible dangers associated with fractured glass.

The applicator of the invention is particularly suitable for use in conjunction with liquid adhesives, sealants and coatings particularly for medical applications. The applicator is particularly suitable for use with liquid cyanoacrylate compounds that polymerise on exposure to air.

The hollow body portion is such that at least the walls of the reservoir portion are of flexible material to allow the actuator means to squeeze the walls of the reservoir portion to effect controlled discharge of liquid therefrom. Preferably the hollow body portion is wholly of a flexible material e.g. polyethylene or polypropylene, both of which have good flexibility and moisture barrier properties. The polyethylene and polypropylene may be of either high or low density

The plug is ideally of a different material to that of the body to reduce the possibility of the plug and body sticking together. The plug may for example be of polyethylene or polypropylene (both of either high or low density), although ideally a different material from that of the body portion. Alternatively the plug may be of an acetal.

It is also envisaged that the body portion and /or the plug may have a low friction surface (at least in their regions which provide the seal) to reduce the potential of sticking and malfunction. The low friction surface may be provided by an additive at the surface such as PTFE in powder or fibre form. Alternatively the surface of the body portion and/or plug may be manufactured of a fluorinated polymer. Thus, for example, the plug or body portion may have a fluorinated surface, such as provided by PTFE. By way of example, the plug may be of an acetal impregnated with PTFE in either a powder or fibre form. Alternatively the plug may be manufactured from PTFE or FEP or other flouro plastics. The provision of fluorinated material at the surface of the body portion and/or plug (at least in their regions which provide the seal) not only reduces the potential of sticking and malfunction but also improves resistance to moisture ingress.

In a preferred embodiment of the invention, the plug has a head that is a sliding fit on the inner surface of the outlet portion and a shank with a first portion that sealingly locates within the mouth of the reservoir portion. The first portion of the plug shank may be provided with "sealing rings" which whilst providing good sealing capability also reduce resistance to movement during the actuation process. Additionally the inner surface of the outlet portion (on which the head of the plug is slideable) is formed with at least one flow channel which extends from the first end of the outlet portion towards the second end. Preferably a plurality of such flow channels are provided (most preferably equally spaced around the inner surface of the outlet portion) and extend parallel to each other, each such channel preferably being linear.

In use of the applicator, operation of the actuator means initially causes the plug to move to the second position. As a result of this movement, the head of the plug moves forward and disengages with the body. With subsequent squeezing of the walls of the reservoir by the actuator means, the liquid may pass out of the reservoir portion and then past the head of the plug *via* the flow channel(s) formed in the inner surface of the outlet portion. Consequently by virtue of the flow channel(s) the liquid can travel from the reservoir portion to the applicator tip assembly for discharge from the applicator device.

In a preferred embodiment of the invention, the actuator means comprises a first actuator formation for causing the plug to move to the second position and a second actuator formation for subsequent squeezing of the walls of the reservoir.

In certain embodiments of the invention, the end of the head of the plug nearest the applicator tip assembly may, if the plug travels sufficiently far, abut against an internal surface of the applicator device. For such cases, the said end of the head is configured to divert flow of liquid from along the flow channels inwardly towards the centre of the outlet portion for subsequent discharge *via* the applicator tip assembly. This configuration may be provided for by castellations or similar channels extending around the periphery of the end of the head. Thus once the head has abutted against the aforementioned internal surface the castellations effectively define ports through which liquid may pass towards the interior of the outlet portion.

Preferably also the first portion of the shank has over a length thereof a cross-section (preferably circular) which sealingly locates in a corresponding cross-section over a length of the mouth of the reservoir portion. Movement of the plug to its second position causes said first portion of the shank to move out of the mouth of the reservoir portion.

For preference, there is a step between said head and said first portion of the shank. In this case the inner surface of the outlet portion of the body is provided with a shoulder that faces away from the reservoir portion and the step of the plug abuts against said shoulder when the plug is in its first position.

It is also preferred that, at its end opposite the head, the shank has a second portion which is of lesser cross-section than said first portion. This second portion may remain located within the mouth of the reservoir when the plug has been moved to its second position so that the liquid is able to flow through a gap defined between the wall of the mouth and this second portion.

For all embodiments of the invention, it is preferred that the mouth of the reservoir portion is formed with walls that converge towards each other in a direction going away from the applicator tip assembly. A portion of the plug (ideally the above mentioned second portion of the shank) locates between these converging walls. Additionally the preferred actuator is provided with at least one (and more preferably two, although more than two may be provided) actuator means which when operated provide an actuating force to the converging walls, the actuating force having a component directed towards the tip assembly. This actuating force effects movement of the plug towards the second end of the outlet formation. The actuator means may be configured to be operated by the fingers and/or thumb of a user of the device and may be such as to provide a mechanical advantage whereby the force applied to the actuator means by the finger(s) and/or thumb of the user results in a higher actuating force applied to the converging wall of the applicator.

Most conveniently, the applicator has a pair of opposed actuator means in the form of wings or levers (herein referred to simply as levers for convenience) each of which is provided with a first actuator formation and a second actuator formation. The levers are preferably biased away from each other and are pivotally mounted on the body of the applicator for movement towards and away from each other. Squeezing of the levers together initially causes the first actuator formations to dislodge the plug. Further squeezing of the levers causes the second actuator formations to engage the walls of the reservoir portion to effect discharge of the liquid.

The first actuator formations may be in the form of prongs whereas the second actuator formations preferably have arcuate surfaces for squeezing the walls of the reservoir portion. Alternatively the first and second actuator formations of any one lever may comprise a continuously curved component with the second actuator formation being of shallower curvature than the first actuator formation.

The invention will be further described by way of example only with reference to the accompanying drawings, in which:
Fig 1 is a plan view of a first embodiment of liquid applicator in accordance with the invention;
Fig 2 is a side view of the liquid applicator shown in Fig 1;
Fig 3 is a sectional view of the liquid applicator taken along the line A-A in Fig 1;
Fig 4 is a sectional view of the liquid applicator taken along the line B-B in Fig 2;
Fig 5 is a sectional view to an enlarged scale of the liquid applicator taken along the line C-C in Fig 1;
Fig 6 is similar to Fig 5 but omits details of the plug;
Fig 7 is a detail to an enlarged scale of a portion of the applicator as illustrated in Fig 4 but showing the plug positioned off its seat;
Fig 8 is similar to Fig 7 but omitting details of the plug;
Fig 9 is a detail to an enlarged scale of the plug of the applicator;
Fig 10 is a plan view of a second embodiment of liquid applicator in accordance with the invention;
Fig 11 is a cross-sectional view of the hollow body of the liquid applicator illustrated in Fig 10;
Fig 12 is a cross-sectional view of part of the applicator tip assembly (but omitting the actual tip) of the applicator illustrated in Fig 10;
Figs 13a and 13b show (to an enlarged scale) respectively a side view of the plug of the applicator assembly and an end view of the plug as seen in the direction of arrow X in Fig 13a;
Figs 14a-e are views (to an enlarged scale) of a portion of the actuator assembly illustrated in Fig 10 with the actuating levers at various positions;
Fig 15 is a view (to a further enlarged scale) of the actuator in the condition illustrated in Fig 14e;
Fig 16 is a cross-sectional view of the hollow body of a third embodiment of liquid applicator in accordance with the invention;
Fig 17 is a detail to an enlarged scale of a region of the hollow body portion shown in Fig 16; and
Figs 18a and 18b are respectively sectional and side views of an applicator tip assembly for use in conjunction with the body portion illustrated in Fig 16.

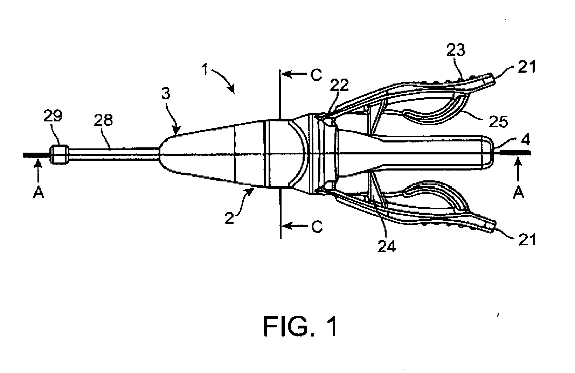

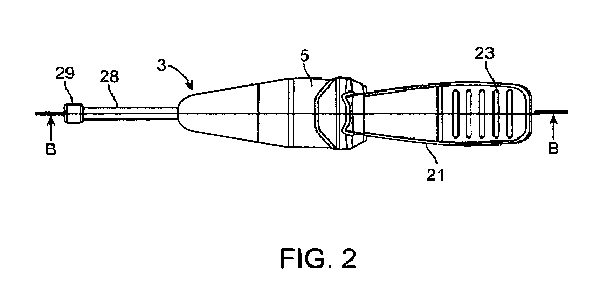

Figs 1-9 of the drawings relate to a first embodiment of liquid applicator 1 that can be used to apply diverse types of fluids. It is particularly well-suited for use with sterilisable fluids for therapeutic applications to mammalian tissue, including topical liquids such as tissue adhesives for surgical applications, coatings and sealants. The liquid may for example be a cyanoacrylate prepolymer and the applicator will be described with specific reference to such a compound (although as indicated it is suitable for other liquids).

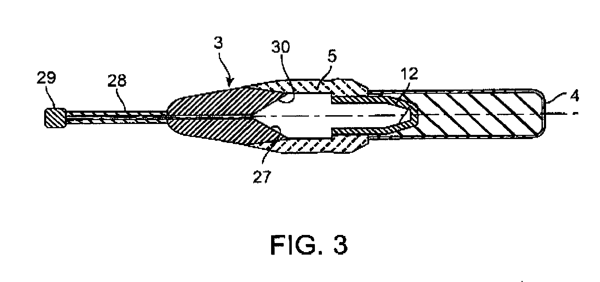

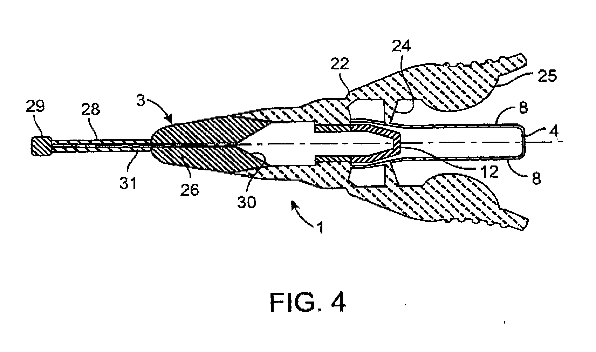

In broad outline, the applicator 1 is for use in applying a fine line of liquid, polymerisable cyanoacrylate adhesive to a skin wound so as to effect closure and/or sealing thereof. The applicator 1 comprises a hollow body 2 on which is mounted an applicator tip assembly 3 (see particularly Figs 3 and 4). The cyanoacrylate adhesive (indicated by cross-hatched lines in Fig 3) is held in a flexible-walled reservoir portion 4 of the applicator body 2 and is discharged when required *via* the applicator tip assembly 3 which is configured to apply the adhesive as the aforementioned fine line. The cyanoacrylate adhesive is retained in the reservoir portion 4 until required by means of a moveable plug 12 (see Figs 3 and 4) and discharge is effected by squeezing together (between the forefinger and thumb of one hand) two levers 21 which are adapted initially to dislodge the plug 12 (to allow liquid flow out of the reservoir portion 4) and then press on walls of the reservoir portion 4 to discharge the cyanoacrylate adhesive through the applicator tip assembly 3.

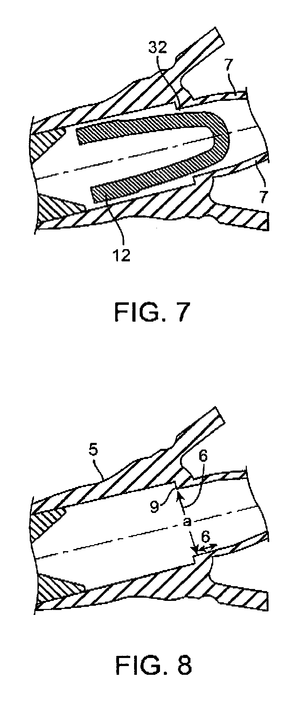

Body portion 2 is moulded from High Density Polyethylene (HDPE) and is formed with the aforementioned reservoir portion 4 which is connected to an outlet portion 5 of the body 2 at a mouth 6 of the reservoir portion 4 (see Fig 8). At its end opposite the reservoir portion 4, the outlet portion 5 serves to locate the applicator tip assembly 3 in position.

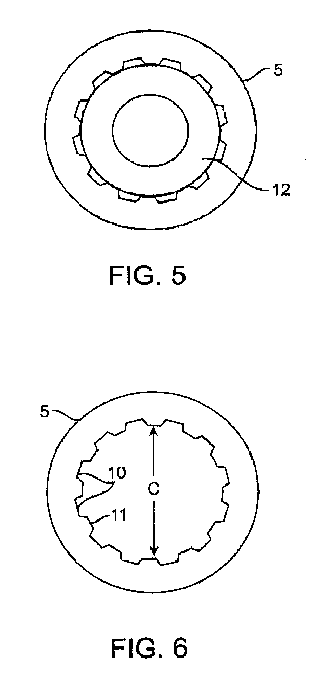

Figs 4 and 6 show that the mouth 6 is defined partly by two opposed walls 7 that converge towards each other in a direction going away from the applicator tip assembly 3 (i.e. the walls converge towards the right hand end of the liquid applicator 1 as viewed in Figs 3 and 4). At the limit at their convergence, the walls 7 are extended as walls 8 that define two opposed sides of the reservoir portion 4. Since the body portion 2 (and thus the reservoir portion 4) is of moulded HDPE, the walls 7 and 8 have a degree of flexibility which is provided for the purposes described more fully below.

Referring now to Fig 8, it will be seen that the inner wall of the outlet portion 5 is formed at its end adjacent the mouth 6 with an annular shoulder 9 which faces towards the applicator tip assembly 3. At the shoulder 9, the mouth 6 is referenced as having a diameter *a* which is constant over a distance *b* going towards the interior of the reservoir portion 4.

A plurality of circumferentially spaced, axially extending grooves 10 which are of generally trapezoidal cross-section (see Figs 5 and 6) are formed in the interior wall of the outlet portion 5 and extend from the shoulder 9 to the applicator tip assembly 3. Expressed alternatively, the inner surface of the outlet portion 5 can in cross-section be seen to comprise alternating trapezoidal section grooves 10 and trapezoidal section ribs 11 (see Figs 5 and 6). The distance between the crests of 2 diametrically opposed ribs 11 is referenced in Fig 6 as *c*. Furthermore the distance that the shoulder 9 projects radially inwardly of the crest of a rib 11 is considered for convenience to be *d.*

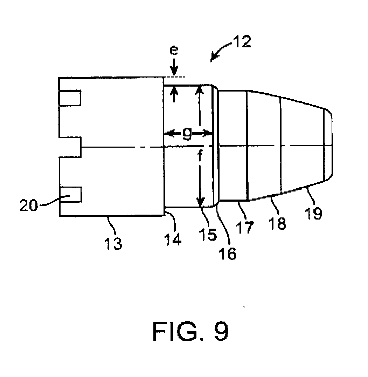

Figs 3 and 4 show the aforementioned plug 12 that serves to retain liquid cyanoacrylate in the reservoir portion 4 and prevent it passing to the outlet portion 5 (and thus to the applicator tip assembly 3) until the plug 12 is dislodged, as described more fully below. Specific detail of the plug 12 is shown in Fig 9 to which is reference is now made. Plug 12 is of moulded polypropylene and (at the left hand end as viewed in Fig 9) has a cylindrical head region 13 having an external diameter (the maximum cross-section for the plug 12) which is the same as *c* (i.e. the distance between the crests of 2 diametrically opposed ribs 11) so as to make the head 13 a close sliding fit on the interior of the outlet portion 5. Going towards the opposite end of the plug 12, there is a shank with the following successive formations, namely:
(i) a radially inwardly directed, right-angled step 14 having a depth *e* which is the same as to the aforementioned distance *d* by which the shoulder 9 projects beyond the crests of the ribs 11;
(ii) a cylindrical, first intermediate region 15 having an external diameter *f* which is the same as the diameter *a* of the mouth 6 of the reservoir portion 4 and a length *g* which is the same as *b*;
(iii) a frustoconical step 16 converging away from the cylindrical region 15;
(iv) a cylindrical, second intermediate region 17;
(v) a tapering third intermediate portion 18; and
(vi) an end portion 19 that tapers at a slightly sharper angle than intermediate portion 18.

A further feature of the plug 12 in the provision of castellations 20 around the free end of head 13. The function of these castellations 20 is described below.

Purely for the purposes of moulding, plug 12 is hollow (to ensure the required tolerances) and is open at the head 13 and closed at the opposite end of tapering portion 19.

Integrally moulded with the exterior of the outlet portion 5 of the body 2, and spaced 180° apart from each other, are the two levers 21. More particularly, each lever 21 is integrally connected to the body portion 5 by a flexible neck 22 such that the levers 21 may be squeezed together but when released resile to their original position. For the purposes of being squeezed together, each lever 21 is provided with a respective finger/thumb grip formation 23, the arrangement being such that the finger/thumb grip formations 23 may be held between the thumb and forefinger of one hand. Each lever has a prong formation 24 which with the levers 21 being in their original position (i.e. without the levers being squeezed together) just comes into contact with a respective wall 7 of the mouth 6 of reservoir portion 4. Each prong formation 24 is angled slightly away from the free end of the respective lever 21 for reasons which will be described more fully below. Each lever 21 additionally formed with two generally arcuate actuator formations 25 which, on squeezing levers 21 together beyond a certain point, come into contact with the walls 8 of reservoir portion 4.

The liquid applicator 1 is completed by the applicator tip assembly 3 which has the structure illustrated in Figs 3 and 4. More particularly, applicator tip assembly 3 has a body 26 formed at one end as a spigot 27 for locating assembly 3 as a push-fit on the free end of outlet portion 5 (the spigot 27 being located in engagement with the interior surface of outlet portion 5) and being provided at the other end with an elongate probe 28 itself provided with a fibrous applicator tip 29 at its free end. Although not shown in Figs 3 and 4, the spigot 27 may have a circumferential ridge for location in a complementary annular groove (again not shown) extending around the inner surface of outlet portion 5. Such a ridge and groove arrangement serves not only to locate the body portion 2 and applicator tip assembly 3 together but also provides for leak free operation of the liquid applicator 1. The end of the spigot 27 converges towards the probe 28 as shown so as to provide a "funnel" 30 which leads into a narrow bore 31 extending along the length of the remainder of the body 26 and throughout the length of the probe 28.

Assembly and use of the liquid applicator 1 will now be described.

Body portion 2 (with an integral levers 21), applicator tip assembly 3 and plug 12 are initially provided as three separate, moulded components.

Prior to assembly of the device, the body portion 2 is positioned vertically (outlet portion 5 uppermost) and the reservoir portion 4 is filled to the required level with liquid cyanoacrylate adhesive to be dispensed by the device.

In the next step of assembly, the tapering end 19 of plug 12 is inserted into the open end of outlet portion 5 and the plug 12 is pushed along the latter portion until its step 14 comes into abutting engagement with the shoulder 9. During this movement, the cylindrical region 15 of the plug 12 enters the mouth 6 and forms a seal over the aforementioned distance *b* so that the liquid cyanoacrylate adhesive is prevented from being discharged.

Applicator tip assembly 3 may now be located as a push-fit on the free end of outlet portion 5 so as to produce the device as illustrated in Figs 1-4.

In order to discharge liquid cyanoacrylate adhesive from the applicator 1, the two levers 21 may be gripped between the thumb and forefinger of one hand and squeezed together. Initially this squeezing together of the levers causes the prongs 24 to act against the walls 7 and apply a force which causes the plug 12 to move to the position illustrated in Fig 7 (i.e. the plug moves to the left from its position shown in Figs 3 and 4) such that its cylindrical region 15 is moved wholly clear of the mouth 6 of the reservoir portion 4. There is now an annular gap 32 between the shoulder 9 (of the outlet portion 5) and the plug 12 which is allowed for by the configuration of its frustoconical step 16 and tapering region 18 and 19. Consequently the interior of the reservoir portion 4 is now in communication (*via* the annular gap 32) with the trapezoidal cross-section channels 10 formed in the inner wall of the outlet portion 5.

Continued squeezing of the levers 21 together causes the arcuate actuators 25 to engage and squeeze the walls 8 of the reservoir portion and cause liquid contained therein to be expressed through the annular gap 32 and along the channels 10. Liquid is therefore able to flow along the channels 10 and into the funnel-section 30 at the spigot end of the applicator tip assembly and then along the narrow bore for discharge *via* the fibrous tip 29. As the levers 21 are squeezed, the adhesive exits the tip 29 and results in a vacuum being created behind the adhesive left in the reservoir portion 4. Once the wings 21 are released, this vacuum draws adhesive back from the tip 29 thereby preventing drips and improving application control. Release of the levers 21 (so that arcuate actuators 25 no longer contact the walls 8) allow the walls 8 to return to their original position and cause a suction force to be generated which causes liquid to be drawn backwardly into the device.

Thus the device is capable of accurately applying only small amounts of liquid and of terminating the application when desired.

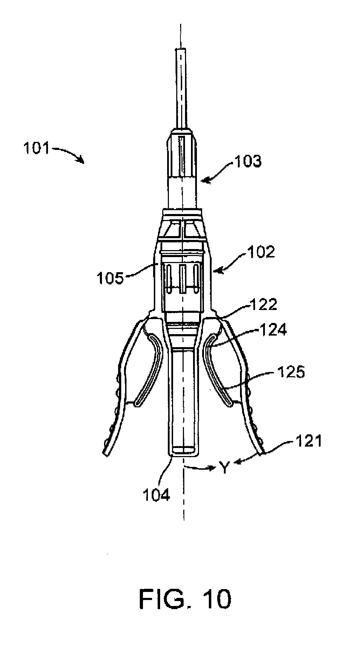

Reference is now made to Figs 10-15 of the drawings which relate to a second embodiment of liquid applicator 101. It will be seen from these figures that the second embodiment of liquid applicator 101 has marked similarity with the first embodiment of liquid applicator 1 described with reference to Figs 1-9. Therefore for the sake of convenience those parts of the applicator 101 which correspond with an equivalent part in the liquid applicator 1 are referenced by a numeral which is one hundred greater than the corresponding reference numeral in Figs 1-9. Thus purely by way of example the body portion of the applicator 101 is denoted by the reference numeral 102 (cf reference numeral 2 used for the body portion of liquid applicator 1).

In view of the similarities between the first embodiment of liquid applicator 1 and second embodiment of liquid applicator 101 only a brief description of the latter will be given so as to highlight certain features.

One particular feature of difference between the first embodiment of liquid applicator 1 and the second embodiment of liquid applicator 101 lies in the first and second actuator formations. In the liquid applicator 1 the first actuator formation 24 is depicted as being a prong and the second actuator formation 25 being arcuate. In contrast, the first and second actuator formations 124 and 125 in the second embodiment of liquid applicator 101 are each formed as part of a continuously curving component with the first actuator formation 124 being of more pronounced curvature than the second actuator formation 125.

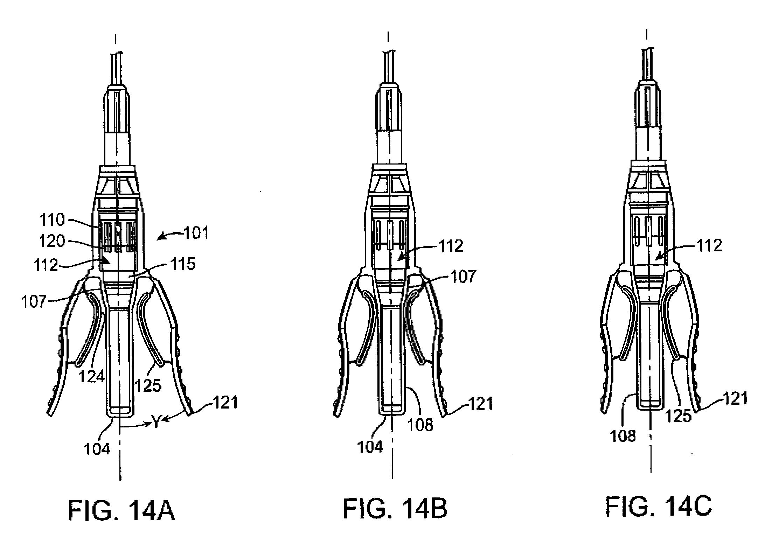

The manner in which the first and second actuator formations 124 and 125 function to effect discharge of the contents of the applicator 101 is described below with reference to Figs 14a-e.

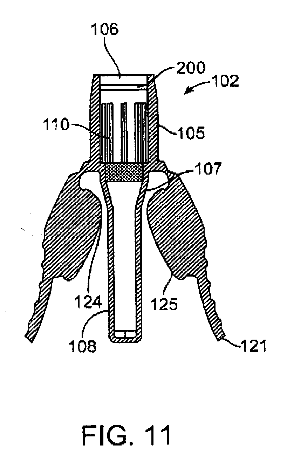

Further detail of the body portion 102 (with its integral levers 121) is seen in the cross-section of Fig 11 which clearly shows (some of) the longitudinal grooves or flow channels 110 that are formed in, and extend in parallel along, the inner wall of the outlet portion 105. Additionally shown in Fig 11 is a circumferential (i.e. annular) groove 200 formed on the inner surface of the outlet portion 105 in a region thereof between its open end and the ends of the grooves 110.

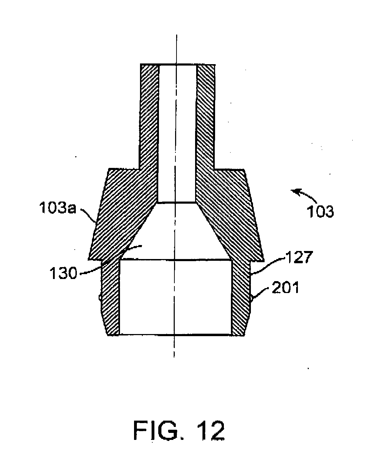

A cross-section of part of the applicator assembly 103 is shown in Fig 12. In fact, in the embodiment of Figs 10-15, the applicator assembly may be in two parts, one of which is shown in Fig 12 referenced as 103a and the other part of which (not shown in Fig 12) is the applicator tip. With such an arrangement, it will be appreciated that different applicator tips may be mounted on part 103a depending on the intended use of the device. With further reference to Fig 12 it will be seen that part 103a includes a circumferential ridge 201 on the spigot 127. It will be appreciated that the circumferential groove 200 on the body portion 102 and the circumferential ridge 201 co-operate during assembly of the applicator 101 so as to locate the body portion 102 and applicator tip assembly 103 together and to provide for a leak free operation.

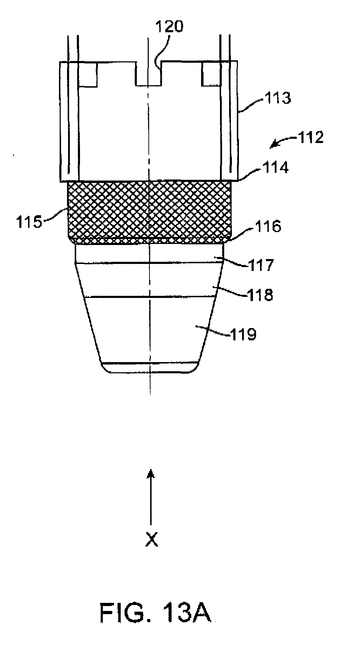

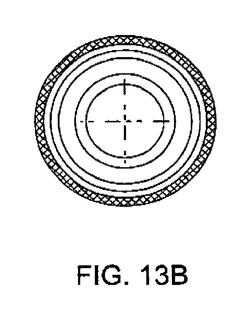

Side and end views of the plug 112 are shown in Figs 13a and 13b respectively. The cylindrical first intermediate region 115 and frustoconical step 116 are shown in Fig 13a with cross-hatched shading. This is to represent the sealing area of the plug which is intended to cooperate with the correspondingly cross-hatched area within the body portion 102 as shown in Fig 11. In the regions of these cross-hatched areas the plug and/or body portion may be specially treated to ensure a leak free seal between the plug and the body to reduce coefficient of friction. In a modified construction (not shown in the drawings) the region 115 of the plug 112 may be provided with sealing rings to prevent egress of liquid (from reservoir 104 until the plug 112 has been dislodged from its seated position.

Reference is now made to Figs 14a-14e which are "see-through" views of the liquid applicator 101 illustrating the location of the plug 112 for various angular positions of the levers 121.

In more detail, Fig 14a shows the situation where the levers 121 are relaxed so that the actuator formations 124 and 125 are out of contact with the reservoir portion 104. Squeezing of the levers 121 together so that they are deflected by 7 degrees from the position illustrated in Fig 14a brings them to the position shown in Fig 14b in which the first actuator formation 124 has just come into contact with the reservoir portion 104 (more particularly the walls 107 thereof).

Movement of the levers 121 by a further 3 degrees (i.e. 10 degrees from the position shown in Fig 14a) brings them to the position shown in Fig 14c in which the first actuator formations 124 have caused the plug 112 to be dislodged slightly (i.e. moved upwardly as viewed in the drawing) as compared to the position in Figs 14a and 14b and the second actuator formations 125 are beginning to act against the walls 108 of the reservoir 104. However at this position the cylindrical, first intermediate region 105 of the plug 112 still locates within the mouth 106 of the reservoir portion 104 so that adhesive is retained within the reservoir portion 104.

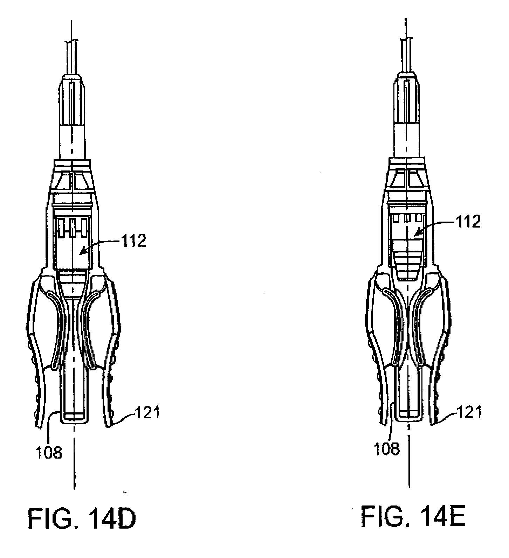

Once the levers 121 have been deflected by 16 degrees from their original position shown in Fig 14a, the condition shown in Fig 14d is reached. At this point the cylindrical, first intermediate region 115 of the plug 112 has moved by a distance g so that it (the first intermediate portion 115) has just moved out of the mouth 106. In fact, if the applicator 101 were turned "upside-down" from the upright position illustrated in Fig 14d then the plug 112 would move vertically down under gravity. It will be noted from Fig 14d that the second actuator formations 125 are now applying significant compressive force to the walls 108 reservoir 104.

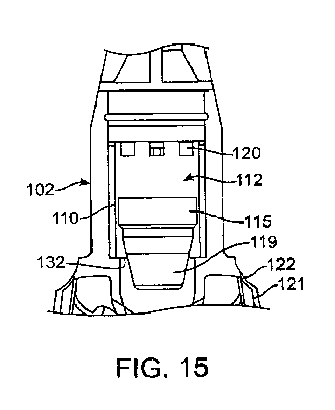

Referring now to Fig 14e, the levers 121 have been moved through a further 14 degrees as compared to the position shown in Fig 14d (and therefore 20 degrees from the initial position shown in Fig 14a). During this further movement, the second actuator formations 125 continue to compress the walls 108 of the reservoir portion 104 causing liquid adhesive to be discharged therefrom. The liquid adhesive is able to flow through the annular gap now formed between the plug 112 and the mouth 106 and then along the flow channels 110 for ultimate discharge from the applicator tip assembly 103. During this further movement of the levers 121 (i.e. from the position shown in Fig 14d to that in Fig14e) the plug 112 moves along the outlet portion 105 of the body 102 until it (the plug 112) abuts against the upstream end of the funnel region 130 of the applicator tip assembly 103. For the purposes of clarity this is shown in the much enlarged view of Fig 15. It will be appreciated that liquid adhesive that has exited the downstream ends of the channels 110 is able to flow into the applicator tip assembly 3 *via* the catastellated formations 120 provided on the plug 12.

The position of the levers 121 as depicted in Fig 14e represents their maximum deflection towards each other. As the levers 121 are released from this position, adhesive is drawn back from the applicator tip 128 (as described previously) thereby preventing drips and improving application control.

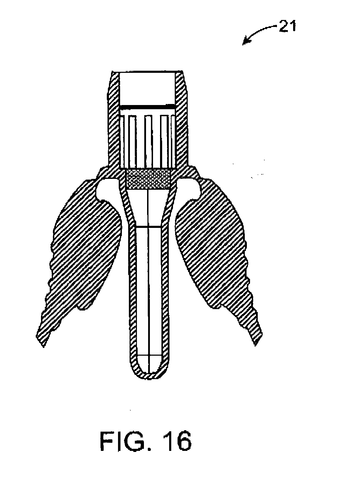

Reference is now made to the third embodiment of liquid applicator as shown in Figs 16-18 of the drawings for which (for convenience) parts corresponding with an equivalent part in the liquid applicator 1 are referenced by a numeral which is 300 greater than the corresponding reference numeral in Figs 1-9.

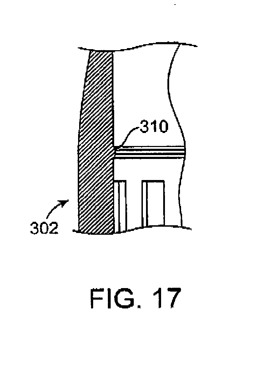

The hollow body portion 302 shown in Fig 16 is very similar to the hollow body portion 102 shown in Fig 11 but has an annular rib 310 (within the hollow body portion) instead of the annular groove 200 (see also the expanded view of Fig 17).

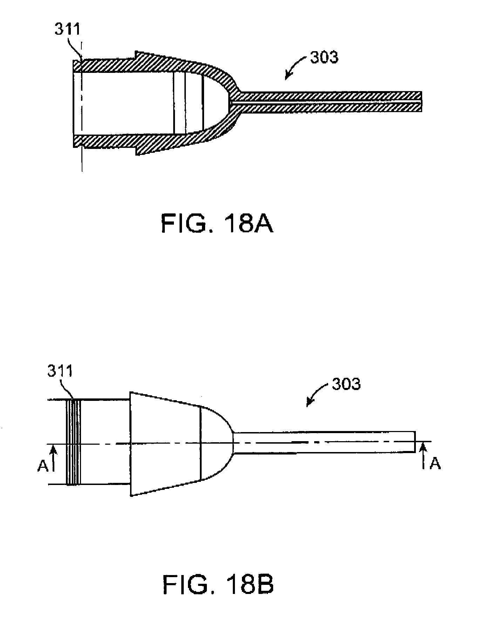

Applicator assembly 303 (Fig 18) incorporates a circumferential groove 311 in which the annular rib 310 is intended to locate whereby the body portion 302 and applicator assembly 303 are secured together.

Examples of cyanoacrylate adhesive compositions that may be incorporated in the applicator are given below.

The adhesive fluids that may be applied by the applicator of the present invention may be comprised of a wide variety of cyanoacrylate adhesive formulations. The reservoir may contain a stronger bonding and less flexible cyanoacrylate adhesive composition, such as *n*-butyl cyanoacrylate, or it may contain a more flexible tissue adhesive, such as an octyl or hexyl or decyl or other homologs of cyanoacrylate.

Preferably, the cyanoacrylate compositions used comprise cyanoacrylate prepolymer compositions that can be applied as a liquid/gel to the skin surface. Optionally, the cyanoacrylate prepolymers can include therapeutic agents such as analgesics, anti-inflammatory agents, antimicrobial agents, and the like.

Preferably, the polymerizable cyanoacrylate prepolymers comprise cyanoacrylate esters that, in monomeric form, are represented by the formula I: 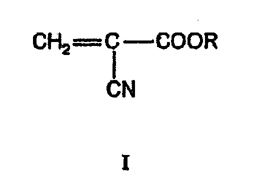 wherein R is selected from the group consisting of:
alky of 1 to 10 carbon atoms,
akenyl of 2 to 10 carbon atoms,
cycloalky groups of from 5 to 8 carbon atoms, phenyl,
2-ethoxyethyl,
3-methoxybutyl,
and a substituent of the formula: 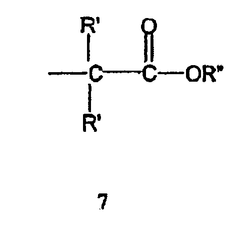 wherein
   each R' is independently selected from the group consisting of:
hydrogen and methyl, and
R" is selected from the group consisting of:
alkyl of from 1 to 6 carbon atoms,
alkenyl of from 2 to 6 carbon atoms,
alkynl of from 2 to 6 carbon atoms,
cycloalkyl of from 3 to 8 carbon atoms,
aralkyl selected from the group consisting of benzyl, methylbenzyl and phenylethyl,
phenyl, and phenyl substituted with 1 to 3 substituents selected from the group consisting of hydroxyl, chloro, bromo, nitro, alkyl of 1 to 4 carbon atoms, and alkoxy of from 1 to 4 carbon atoms.

More preferably, in the cyanoacrylate esters of formula I, R is an alkyl group of from 2 to 10 carbon atom including ethyl, *n*-propyl, iso-propyl, *n*-butyl, iso-butyl, sec-butyl, n-pentyl, iso-pentyl, n-hexyl, 2-ethylhexyl, n-heptyl, octyl, nonyl, and decyl. Mixtures of such compounds can also be employed as disclosed by Berger, et al., U.S. Patent No. 5,998,472.

It is to be understood that the term "polymerizable cyanoacrylate esters" refers to polymerizable formulations comprising cyanoacrylate monomers or polymerizable oligomers which, in their monomeric form, are preferably compounds represented by formula I as described above.

The polymerizable cyanoacrylate esters described herein rapidly polymerize in the presence of water vapour or tissue protein, and the n-butyl-cyanoacrylate bonds to mammalian skin tissue without causing histotoxicity or cytotoxicity.

Polymerizable cyanoacrylate esters are known in the art and are described in, for example, U.S. Patent Nos. 3,527,224; 3,591,676; 3,667,472; 3,995,641; 4,035,334; and 4,650,826 .

Optionally, the cyanoacrylate compositions applied by the present applicator can include a "biocompatible plasticizer". As used herein, the "biocompatible plasticizer" refers to any material which is soluble or dispersible in the cyanoacrylate composition, which increases the flexibility of the resulting polymeric film coating on the skin surface, and which, in the amounts employed, its compatible with the skin as measured by the lack of moderate to severe skin irritation. Suitable plasticizers are well known in the art and include those disclosed in U.S. Patent Nos. 2,784,127 and 4,444,933 . Specific plasticizers include, by way of example only, acetyl tri-n-butyl citrate (preferably ∼20 weight percent or less), acetyl trihexyl citrate (preferably ∼ 20 weight percent or less) butyl benzyl phthalate, dibutyl phthalate, dioctylphthalate, n-butyryl tri-n-hexyl citrate, diethylene glycol dibenzoate (preferably ∼20 weight percent or less) and the like. The particular biocompatible plasticizer employed is not critical and preferred plasticizers include dioctylphthalate and C₂-C₄-acyl tri-n-hexyl citrates.

Optionally as well, the cyanoacrylate composition applied by the present applicator can include an "antimicrobial agent". As used herein, the term "antimicrobial agent" refers to agents which destroy microbes (i.e. bacteria, fungi, yeasts and viruses) thereby preventing their development and their pathogenic action.

Preferred cyanoacrylate compositions useful in the practice of this invention are also disclosed by Greff, et al., U.S. Patent No. 5,480,935 . In a particularly preferred embodiment, the cyanoacrylate adhesive composition further comprises an antimicrobially effective amount of compatible antimicrobial agent. Such compositions preferably comprise from 0.1 to about 30 and preferably about 0.5 to 10 weight percent of the compatible antimicrobial agent either as a solution or as suspension based on the total weight of the composition. Compatible antimicrobial agents are those which are either soluble or suspendable in the cyanoacrylate composition, which do not cause premature polymerization of the cyanoacrylate composition, which do not prevent polymerization of the cyanoacrylate composition when applied to mammalian skin, and which are compatible with the intended use including biocompatibility with the patient's skin. Suitable such compositions of cyanoacrylate/povidone-iodine complexes, and U.S. Patent Application Serial No. 60/498,913 filed on August 29, 2003, which discloses compositions of cyanoacrylate esters and phenol.

The use of compatible antimicrobial agent in the compositions permits the agent to be released from the polymeric film thereby reducing microbial growth adjacent to the film.

Other medicaments suitable for use in conjunction with the cyanoacrylate compositions include corticoid steroids such as described by Greff, et al. in U.S. Patent No. 5,962,010 and analgesic compounds such as lidocaine. The former reduces inflammation whereas the latter reduces pain. Combinations of a steroid with an analgesic are also covered.

## Claims

1. A liquid applicator (1,101) comprising:
(a) a hollow body portion (2,102) incorporating (i) a reservoir portion (4,104) for containing a liquid to be applied by the applicator, and (ii) an outlet portion (5,105) having first and second ends, the first end being connected to an outlet mouth (6,106) of the reservoir portion (4,104),
(b) an applicator tip assembly (3,103) provided on the second end of the outlet portion (5,105) for discharge of liquid from the reservoir portion (4,104) *via* said outlet portion,
(c) a plug (12,112) located in the mouth of the reservoir portion (4,104), and
(d) actuator means (21,121) for squeezing the walls (8,108) of the reservoir portion to effect discharge of liquid from the reservoir portion *via* the outlet portion (5,105) and the applicator tip assembly (3,103), wherein
said plug (12,112) and mouth are configured so that the plug is moveable towards the applicator tip assembly (3,103) from a first position at which it seals the reservoir portion (4,104) to a second position at which fluid may follow out of the reservoir portion, **characterised in that** the actuator means (21,121) are adapted to apply an actuating force to the mouth wall (7,107) to effect movement of the plug from its first position to the second position.

2. An applicator (1,101) as claimed in claim 1 wherein the inner surface of the outlet portion (5,105) is formed with at least one channel (10,110) that extends from the first end of the outlet portion towards the second end, the plug (12,112) has a head (13,113) that is a sliding fit on the inner surface of the outlet portion (5,105) and a shank with a first portion (15,115) that sealingly locates within the mouth of the reservoir portion (4,104), the arrangement being such that movement of the plug (12,112) from the first to the second position allows fluid to flow from the reservoir portion (4,104) through said at least one channel (10,110) to the applicator tip assembly (3,103).

3. An applicator (1,101) as claimed in claim 2 wherein a plurality of said channels (10,110) are provided and extend parallel to each other, said channels (10,110) preferably being linear.

4. An applicator (1,101) as claimed in claim 2 or 3 wherein the end of the head (13,113) of the plug (12,112) nearest the applicator tip assembly (3,103) has a configuration to divert flow of liquid from along the flow channels (10,110) inwardly towards the centre of the outlet portion (5,105) for subsequent discharge *via* the applicator tip assembly (3,103), said configuration preferably being provided by castellations (20,120) extending around the periphery of said end of the head (13,113).

5. An applicator (1,101) as claimed in any one of claims 2 to 4 wherein said first portion (15,115) of the shank has over a length (g) thereof a cross-section (f) which sealingly locates in a corresponding cross-section (a) over a length (b) of the mouth of the reservoir portion (4,104) and movement of the plug (12,112) to its second position causes said first portion (15,115) of the shank to move out of the mouth of the réservoir portion (4,104).

6. An applicator (1,101) as claimed in claim 5 wherein, at its end opposite the head (13,113), the plug (12,112) has a second portion (19,119) which is of Lesser cross-section than said first portion (15,115).

7. An applicator (1,101) as claimed in any one of claims 2 to 6 wherein there is a step (14,114) between said head (13,113) and said first portion (15,115) of the shank, the inner surface of the outlet portion (5,105) of the body is provided with a shoulder (9) that faces away from the reservoir portion (4,104) and the step (14,114) of the plug (12,112) abuts against said shoulder (9) when the plug is in its first position.

8. An applicator (1,101) as claimed in any one of claims 1 to 7 wherein the actuator means (21,121) comprises a first actuator formation (24,124) for applying an actuating force to the mouth wall (7,107) to effect movement of the plug (12,112) towards the outlet portion (5,105), and a second actuator formation (25,125) for squeezing the walls (8,108) of the reservoir portion (4,104) to effect discharge of liquid from the reservoir portion (4,104) *via* the outlet portion (5,105) and the applicator tip assembly (3,103).

9. An applicator (1,101) as claimed in claim 8 wherein the mouth of the reservoir portion (4,104) is formed with two opposed walls (7,107) that converge towards each other in a direction going away from the applicator tip assembly (3,103), and the applicator (1,101) is provided with two said first actuator formations (24,124) each of which is adapted to apply a actuating force to a respective one of said opposed converging walls (7,107) to effect movement of the plug (12,112) towards the second end of the outlet formation (5,105).

10. An applicator (1,101) as claimed in claim 9 having a pair of opposed levers (21,121) each of which is provided with a respective one of said first actuator formations (24,124), said levers (21,121) being moveable towards each other to cause the first actuator formations (24,124) to apply the actuating force to effect movement of the plug (12,112) toward the second end of the outlet portion (5,105), said first actuator formations preferably being in the form of prongs (24,124).

11. An applicator (1,101) as claimed in claim 10 wherein the levers (21,121) are each provided with a said second actuator formation (25,125) and said second actuator formations have arcuate surfaces for squeezing the walls (8,108) of the reservoir portion (4,104).

12. An applicator (1,101) as claimed in claim 10 or 11 wherein the levers (21,121) are biased away from each other.

13. An applicator (1,101) as claimed in any one of claims 10 to 12 wherein an end of each lever (21,121) is pivotally mounted on the exterior of the outlet portion (5,105) in the region of the first end thereof.

14. An applicator (1,101) as claimed in any one of claims 1 to 13 wherein the body is of high density polyethylene and the plug (12,112) is of polypropylene.

15. An applicator (1,101) as claimed in any one of claims 1 to 14 containing a liquid, e.g. a medical adhesive, sealant or coating, preferably a cyanoacrylate prepolymer.

## Patentansprüche

1. Flüssigkeitsapplikator (1, 101), der aufweist:
(a) einen hohlen Körperabschnitt (2, 102), der (i) einen Vorratsbehälterabschnitt (4, 104) für das Aufnehmen einer vom Applikator anzuwendenden Flüssigkeit und (ii) einen Austrittsabschnitt (5, 105) mit einem ersten und zweiten Ende enthält, wobei das erste Ende mit einer Austrittsöffnung (6, 106) des Vorratsbehälterabschnittes (4, 104) verbunden ist;
(b) eine Applikatorkopfbaugruppe (3, 103), die am zweiten Ende des Austrittsabschnittes (505) für das Entleeren der Flüssigkeit aus dem Vorratsbehälterabschnitt (4, 104) über den Austrittsabschnitt vorhanden ist;
(c) einen Stopfen (12, 112), der in der Öffnung des Vorratsbehälterabschnittes (4, 104) angeordnet ist; und
(d) ein Betätigungsmittel (21, 121) für das Zusammendrücken der Wände (8, 108) des Vorratsbehälterabschnittes, um ein Entleeren der Flüssigkeit aus dem Vorratsbehälterabschnitt über den Austrittsabschnitt (5, 105) und die Applikatorkopfbaugruppe (3, 103) zu bewirken, wobei
der Stopfen (12, 112) und die Öffnung so ausgebildet sind, dass der Stopfen in Richtung der Applikatorkopfbaugruppe (3, 103) aus einer ersten Position, in der er den Vorratsbehälterabschnitt (4, 104) abdichtet, in eine zweite Position beweglich ist, in der das Fluid aus dem Vorratsbehälterabschnitt gelangen kann, **dadurch gekennzeichnet, dass** das Betätigungsmittel (21, 121) ausgebildet ist, um eine Betätigungskraft auf die Öffnungswand (7, 107) anzuwenden, um die Bewegung des Stopfens aus seiner ersten Position in die zweite Position zu bewirken.

2. Applikator (1, 101) nach Anspruch 1, bei dem die Innenfläche des Austrittsabschnittes (5, 105) mit mindestens einem Kanal (10, 110) ausgebildet ist, der sich vom ersten Ende des Austrittsabschnittes in Richtung des zweiten Endes erstreckt, wobei der Stopfen (12, 112) einen Kopf (13, 113), der eine Gleitpassung auf der Innenfläche des Austrittsabschnittes (5, 105) aufweist, und einen Schaft mit einem ersten Abschnitt (15, 115) aufweist, der sich abdichtend innerhalb der Öffnung des Vorratsbehälterabschnittes (4, 104) anordnet, wobei die Anordnung so ist, dass die Bewegung des Stopfens (12, 112) aus der ersten in die zweite Position gestattet, dass das Fluid aus dem Vorratsbehälterabschnitt (4, 104) durch den mindestens einen Kanal (10, 110) zur Applikatorkopfbaugruppe (3, 103) fließt.

3. Applikator (1, 101) nach Anspruch 2, bei dem mehrere der Kanäle (10, 110) vorhanden sind und sich parallel zueinander erstrecken, wobei die Kanäle (10, 110) vorzugsweise linear sind.

4. Applikator (1, 101) nach Anspruch 2 oder 3, bei dem das Ende des Kopfes (13, 113) des Stopfens (12, 112), das der Applikatorkopfbaugruppe (3, 103) am nächsten ist, eine Konfiguration aufweist, um den Flüssigkeitsstrom von entlang den Strömungskanälen (10, 110) nach innen in Richtung der Mitte des Austrittsabschnittes (5, 105) für ein anschließendes Entleeren über die Applikatorkopfbaugruppe (3, 103) abzuleiten, wobei die Konfiguration vorzugsweise durch Verzahnungen (20, 120) bereitgestellt wird, die sich um den Umfang des Endes des Kopfes (13, 113) erstrecken.

5. Applikator (1, 101) nach einem der Ansprüche 2 bis 4, bei dem der erste Abschnitt (15, 115) des Schaftes über eine Länge (g) davon einen Querschnitt (f) aufweist, der sich abdichtend in einem entsprechenden Querschnitt (a) über eine Länge (b) der Öffnung des Vorratsbehälterabschnittes (4, 104) anordnet, und wobei die Bewegung des Stopfens (12, 112) in seine zweite Position bewirkt, dass sich der erste Abschnitt (15, 115) des Schaftes aus der Öffnung des Vorratsbehälterabschnittes (4, 104) heraus bewegt.

6. Applikator (1, 101) nach Anspruch 5, bei dem an seinem Ende entgegengesetzt dem Kopf (13, 113) der Stopfen (12, 112) einen zweiten Abschnitt (19, 119) aufweist, der einen kleineren Querschnitt aufweist als der erste Abschnitt (15, 115).

7. Applikator (1, 101) nach einem der Ansprüche 2 bis 6, bei dem ein Absatz (14, 114) zwischen dem Kopf (13, 113) und dem ersten Abschnitt (15, 115) des Schaftes vorhanden ist, wobei die Innenfläche des Austrittsabschnittes (5, 105) des Körpers mit einem Vorsprung (9) versehen ist, der weg vom Vorratsbehälterabschnitt (4, 104) liegt, und wobei der Absatz (14, 114) des Stopfens (12, 112) an den Vorsprung (9) anstößt, wenn sich der Stopfen in seiner ersten Position befindet.

8. Applikator (1, 101) nach einem der Ansprüche 1 bis 7, bei dem das Betätigungmittel (21, 121) eine erste Betätigungsausbildung (24, 124) für das Anwenden einer Betätigungskraft auf die Öffnungswand (7, 107), um eine Bewegung des Stopfens (12, 112) in Richtung des Austrittsabschnittes (5, 105) zu bewirken, und eine zweite Betätigungsausbildung (25, 125) für das Zusammendrücken der Wände (8, 108) des Vorratsbehälterabschnittes (4, 104) aufweist, um ein Entleeren der Flüssigkeit aus dem Vorratsbehälterabschnitt (4, 104) über den Austrittsabschnitt (5, 105) und die Applikatorkopfbaugruppe (3, 103) zu bewirken.

9. Applikator (1, 101) nach Anspruch 8, bei dem die Öffnung des Vorratsbehälterabschnittes (4, 104) mit zwei gegenüberliegenden Wänden (7, 107) ausgebildet ist, die in Richtung zueinander in einer Richtung konvergieren, die von der Applikatorkopfbaugruppe (3, 103) weg geht, und wobei der Applikator (1, 101) mit zwei ersten Betätigungselementausbildungen (24, 124) versehen ist, von denen eine jede ausgebildet ist, um eine Betätigungskraft auf eine jeweilige der gegenüberliegenden konvergierenden Wände (7, 107) anzuwenden, um eine Bewegung des Stopfens (12, 112) in Richtung des zweiten Endes der Austrittsausbildung (5, 105) zu bewirken.

10. Applikator (1, 101) nach Anspruch 9, der ein Paar gegenüberliegende Hebel (21, 121) aufweist, von denen ein jeder mit einer jeweiligen der ersten Betätigungsausbildungen (24, 124) versehen ist, wobei die Hebel (21, 121) in Richtung zueinander beweglich sind, um zu veranlassen, dass die ersten Betätigungsausbildungen (24, 124) die Betätigungskraft anwenden, um eine Bewegung des Stopfens (12, 112) in Richtung des zweiten Endes des Austrittsabschnittes (5, 105) zu bewirken, wobei die ersten Betätigungselementausbildungen vorzugsweise in der Form von Gabeln (24, 124) vorliegen.

11. Applikator (1, 101) nach Anspruch 10, bei dem die Hebel (21, 121) jeweils mit einer zweiten Betätigungsausbildung (25, 125) versehen sind, und wobei die zweiten Betätigungsausbildungen bogenartige Flächen für das Zusammendrücken der Wände (8, 108) des Vorratsbehälterabschnittes (4, 104) aufweisen.

12. Applikator (1, 101) nach Anspruch 10 oder 11, bei dem die Hebel (21, 121) voneinander weg vorgespannt sind.

13. Applikator (1, 101) nach einem der Ansprüche 10 bis 12, bei dem ein Ende eines jeden Hebels (21, 121) drehbar auf der Außenseite des Austrittsabschnittes (5, 105) im Bereich von dessen erstem Ende montiert ist.

14. Applikator (1, 101) nach einem der Ansprüche 1 bis 13, bei dem der Körper aus Polyethylen hoher Dichte und der Stopfen (12, 112) aus Polypropylen besteht.

15. Applikator (1, 101) nach einem der Ansprüche 1 bis 14, der eine Flüssigkeit enthält, beispielsweise einen medizinischen Kleber, ein Dichtungsmittel oder eine Beschichtung, vorzugsweise ein Cyanacrylat-Vorpolymer.

## Revendications

1. Applicateur de liquide (1, 101), comprenant :
(a) une partie de corps creux (2, 102), incorporant (i) une partie de réservoir (4, 104) destinée à contenir un liquide devant être appliqué par l'applicateur, et (ii) une partie de sortie (5, 105), comportant des première et deuxième extrémités, la première extrémité étant connectée à une embouchure de sortie (6, 106) de la partie de réservoir (4, 104) ;
(b) un assemblage de pointe d'applicateur (3, 103), agencé sur la deuxième extrémité de la partie de sortie (5, 105) pour décharger le liquide à partir de la partie de réservoir (4, 104) à travers ladite partie de sortie ;
(c) un bouchon (12, 112) agencé dans l'embouchure de la partie de réservoir (4, 104) ; et
(d) des moyens d'actionnement (21, 121) pour serrer les parois (8, 108) de la partie de réservoir, en vue d'entraîner la décharge de liquide à partir de la partie de réservoir à travers la partie de sortie (5, 105) et de l'assemblage de pointe de l'applicateur (3, 103) ; dans lequel
ledit bouchon (12, 112) et ladite embouchure sont configurés de sore que le bouchon peut être déplacé vers l'assemblage de pointe de l'applicateur (3, 103), d'une première position dans laquelle il ferme de manière étanche la partie de réservoir (4, 104), vers une deuxième position, dans laquelle le fluide peut s'écouler hors de la partie de réservoir, **caractérisé en ce que** les moyens d'actionnement (21, 121) sont adaptés pour appliquer une force d'actionnement à la paroi de l'embouchure (7, 107) pour entraîner le déplacement du bouchon de sa première position vers la deuxième position.

2. Applicateur (1, 101) selon la revendication 1, dans lequel la surface interne de la partie de sortie (5, 105) comporte au moins un canal (10, 110), s'étendant de la première extrémité de la partie de sortie vers la deuxième extrémité, le bouchon (12, 112) comportant une tête (13, 113) ajustée par glissement sur la surface interne de la partie de sortie (5, 105), et une tige avec une première partie (15, 115) agencée de manière étance dans l'embouchure de la partie de réservoir (4, 104), l'agencement étant tel que le déplacement du bouchon (12, 112) de la première position vers la deuxième position permet l'écoulement du fluide de la partie de réservoir (4, 104) à travers ledit au moins un canal (10, 110), vers l'assemblage de pointe de l'applicateur (3, 103).

3. Applicateur (1, 101) selon la revendication 2, comportant plusieurs desdits canaux (10, 110), s'étendant parallèlement les uns aux autres, lesdits canaux (10, 110) étant de préférence linéaires.

4. Applicateur (1, 101) selon les revendications 2 ou 3, dans lequel l'extrémité de la tête (13, 113) du bouchon (12, 112) la plus proche de l'assemblage de pointe de l'applicateur (3, 103) a une configuration telle à dévier l'écoulement de liquide s'écoulant le long des canaux d'écoulement (10, 110) vers l'intérieur, en direction du centre de la partie de sortie (5, 105), en vue d'une décharge ultérieure à travers l'assemblage de pointe de l'applicateur (3, 103), ladite configuration étant de préférence établie par des créneaux (20, 120), s'étendant autour de la périphérie de ladite extrémité de la tête (13, 113).

5. Applicateur (1, 101) selon l'une quelconque des revendications 2 à 4, dans lequel ladite première partie (15, 115) de la tige présente sur sa longueur (g) une section transversale (f) positionnée de manière étanche dans une section transversale correspondante (a) sur une longueur (b) de l'embouchure de la partie réservoir (4, 104), le déplacement du bouchon (12, 112) vers sa deuxième position entraînant le déplacement de ladite première partie (15, 115) de la tige hors de l'embouchure de la partie de réservoir (4, 104).

6. Applicateur (1, 101) selon la revendication 5, dans lequel, au niveau de son extrémité opposée à la tête (13, 113), le bouchon (12, 112) comporte une deuxième partie (19, 119) ayant une section transversale plus petite que ladite première partie (15, 115).

7. Applicateur (1, 101) selon l'une quelconque des revendications 2 à 6, dans lequel un gradin (14, 114) est formé entre ladite tête (13, 113) et ladite première partie (15, 115) de la tige, la surface interne de la partie de sortie (5, 105) du corps comportant un épaulement (9), orienté à l'écart de la partie de réservoir (4, 104), le gradin (14, 114) du bouchon (12, 112) butant contre ledit épaulement (9) lorsque le bouchon se trouve dans sa première position.

8. Applicateur (1, 101) selon l'une quelconque des revendications 1 à 7, dans lequel le moyen d'actionnement (21, 121) comprend une première structure d'actionnement (24, 124) pour appliquer une force d'actionnement à la paroi de l'embouchure (7, 107), afin d'entraîner un déplacement du bouchon (12, 112) vers la partie de sortie (5, 105), et une deuxième structure d'actionnement (25, 125), pour serrer les parois (8, 108) de la partie de réservoir (4, 104), afin d'entraîner la décharge de liquide de la partie de réservoir (4, 104) à travers la partie de sortie (5, 105) et l'assemblage de pointe de l'applicateur (3, 103).

9. Applicateur (1, 101) selon la revendication 8, dans lequel l'embouchure de la partie de réservoir (4, 104) comporte deux parois opposées (7, 107) convergeant l'une vers l'autre dans une direction allant à l'écart de l'assemblage de pointe de l'applicateur (3, 103), l'applicateur (1, 101) comportant deux dites structures d'actionnement (24, 124), chacune étant adaptée pour appliquer une force d'actionnement à une paroi respective desdites parois convergentes opposées (7, 107), pour entraîner le déplacement du bouchon (12, 112) vers la deuxième extrémité de la forme de sortie (5, 105).

10. Applicateur (1, 101) selon la revendication 9, comportant une paire de leviers opposés (21, 121), comportant chacun une structure respective desdites premières structures d'actionnement (24, 124), lesdits leviers (21, 121) pouvant être déplacés l'un vers l'autre pour entraîner las premières structures d'actionnement (24, 124) à appliquer la force d'actionnement, pour entraîner le déplacement du bouchon (12, 112) vers la deuxième extrémité de la partie de sortie (5, 105), lesdites premières structures d'actionnement ayant de préférence la forme de fourchons (24, 124).

11. Applicateur (1, 101) selon la revendication 10, dans lequel les leviers (21, 121) comportent chacun une deuxième dite structure d'actionnement (25, 125), lesdites deuxièmes structures d'actionnement comportant des surfaces arquées pour serrer les parois (8, 108) de la partie de réservoir (4, 104).

12. Applicateur (1, 101) selon les revendications 10 ou 11, dans lequel les leviers (21, 121) sont poussés à l'écart l'un de l'autre.

13. Applicateur (1, 101) selon l'une quelconque des revendications 10 à 12, dans lequel une extrémité de chaque levier (21, 121) est montée de manière pivotante sur l'extérieur de la partie de sortie (5, 105), dans la région de sa première extrémité.

14. Applicateur (1, 101) selon l'une quelconque des revendications 1 à 13, dans lequel le corps est composé de polyéthylène haute densité, le bouchon (12, 112) étant composé de polypropylène.

15. Applicateur (1, 101) selon l'une quelconque des revendications 1 à 14, contenant un liquide, par exemple un adhésif médical, un produit d'étanchéité ou un revêtement, de préférence un prépolymère de cyanoacrylate.
